# EUROPEAN PATENT SPECIFICATION

(11) **EP 1 244 487 B1**
(45) Date of publication and mention of the grant of the patent: **11.10.2006**
(21) Application number: 01900382.1
(22) Date of filing: 02.01.2001
(51) Int. Cl.: A61M 15/00

(54) **AEROSOL INHALER**
AEROSOLINHALATOR
INHALATEUR A AEROSOL

(30) Priority: 07.01.2000 IT MI000010
(43) Date of publication of application: 02.10.2002
(73) Proprietor: CHIESI FARMACEUTICI S.p.A., I-43100 Parma (IT)
(72) Inventor: BRAMBILLA, Gaetano, I-43100 Parma (IT); PANZA, Isabella, I-43100 Parma (IT); FERRARIS, Alessandra, I-43100 Parma (IT)
(74) Representative: Minoja, Fabrizio
(86) International application number: PCT/EP2001/000002
(87) International publication number: WO 2001/049350

(56) References cited:
- EP-A- 0 911 048
- WO-A-92/20391
- WO-A-98/03533
- WO-A-99/12596

## Description

Pressurized metered dose inhalers are well known devices for administering pharmaceutical products to the respiratory tract by inhalation.

Active materials commonly delivered by inhalation include bronchodilators such as β2 agonists and anticholinergics, corticosteroids, anti-leukotrienes, anti-allergics and other drugs that may be efficiently administered by inhalation, thus increasing the therapeutic index and reducing the side effects thereof.

Notwithstanding their apparent simplicity, common pressurized cans for dispensing metered doses of aerosol are difficult to use correctly, as is confirmed by much scientific literature which states that most patients use them incorrectly either because they are unable to synchronize pressing the can with inhaling and hence do not inhale the medicament at the correct time or because they do not maintain an adequate inhalatory flow rate or do not inhale deep enough, among other reasons.

This problem becomes even more important in the case of certain patients such as children, the elderly and patients with reduced respiratory or manual capability.

Even if a dispensing can for aerosol medicaments is used correctly, the availability of an inhaled medicament to the airways largely depends on the size of the aerosol droplets, which in its turn depends on the formulation and on the propellant evaporation time.

It is well documented that even under the most favorable conditions only 10% of the aerosol dose delivered by a pressurized can reaches the respiratory tract. A similar percentage is expired or is deposited outside the oral cavity, whereas because of the impact of the high speed particles about 80% is deposited within the oropharyngeal cavity, swallowed and systemically absorbed and hence practically lost.

The quantity of medicament inhaled is however usually sufficient to achieve the pharmacological effect. However, if the pressurized can is not used properly, the quantity of medicament which reaches the action site at the pulmonary level is further reduced and the therapeutic response is compromised.

Excessive aerosol deposition in the oropharyngeal cavity can also lead to undesirable effects either at the systemic level, as a consequence of the drug absorption, or at the local level, as is the case with corticosteroids, which can result in oral candidiasis. In an attempt to overcome the problems connected with the use of cans releasing metered doses of aerosol medicament, auxiliary delivery systems have been developed over the last decade for application to the nozzles of pressurized dispensing cans which, depending on their shape and size, can be classified as either "spacers" or "reservoirs".

Among reservoirs, Volumatic (Glaxo-Wellcome) is one of the most known and used, while Aerochamber (3M) is one of the most used and known among spacers or small-size auxiliary devices.

In European patent EP-B-0475257, the applicant disclosed a mouth-inhaling device for use with pressurized cans for dispensing metered doses of medicament. This device is highly efficient and has reduced size, so that it can easily be contained in a small bag or even in the pocket of a jacket.

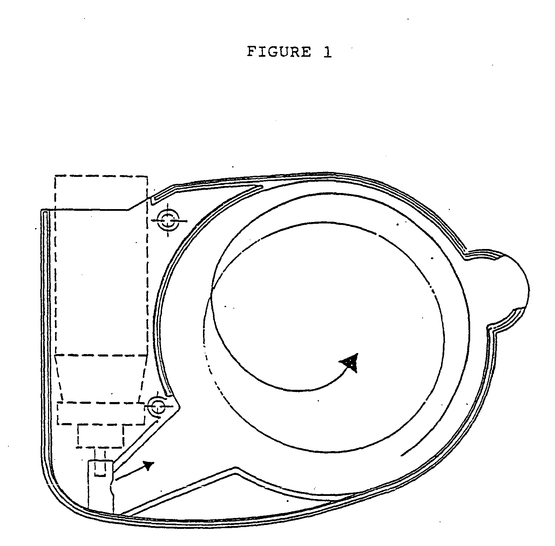

This device (shown in Figure 1) mainly consists of a flat body with a seat tor housing a can, provided with an inhalation mouthpiece and an expansion chamber shaped to create, by virtue of the speed at which the aerosolized material is expelled by the dispenser, a vortex flow in which the particles remain suspended for sufficient time for them to discharge their kinetic energy and allow substantial evaporation of the propellant, with a consequent reduction in the size of the particles, leading to more efficient intrapulmonary and intrabronchial deliveries, while large size particles are centrifuged onto the chamber walls, to deposit on them.

Metered dose inhalers use a propellant to expel droplets containing the pharmaceutical product to the respiratory tract as an aerosol.

For many years the preferred propellants used in aerosols for pharmaceuticals have been a group of chlorofluorocarbons which are commonly called Freons or CFCs, such as CCl₃F (Freon 11 or CFC-11), CCl₂F₂ (Freon 12 or CFC-12), and CClF₂-CClF₂ (Freon 114 or CFC-114).

The device disclosed in EP-B-0475257, marketed under the name Jet, has up to now been used to deliver aerosol medicaments in the form of chlorofluorocarbon suspensions.

Recently, the chlorofluorocarbon (CFC) propellants such as Freon 11 and Freon 12 have been involved in the destruction of the ozone layer and their production is being phased out.

Hydrofluoroalkanes (HFAs), also known as hydrofluorocarbons (HFCs), contain no chlorine, are considered less destructive to ozone and have been proposed as substitutes for CFCs.

HFAs and in particular 1,1,1,2-tetrafluoroethane (HFA 134a) and 1,1,1,2,3,3,3-heptafluoropropane (HFA 227) have been acknowledged to be the best candidates for non-CFC propellants and a number of medicinal aerosol formulations using such HFA propellant systems have been disclosed in many patent applications.

In the international application WO-A-9856349 filed on 10/06/98 the applicant disclosed solution compositions for use in an aerosol inhaler, comprising an active material, a propellant containing a hydrofluoroalkane (HFA), preferably 134a or 227 or mixtures thereof, a cosolvent, preferably ethanol, and further comprising a low-volatility component preferably selected from glycerol, propylene glycol, polyethylene glycol, oleic acid and isopropyl myristate, to increase the mass median aerodynamic diameter (MMAD) of the aerosol particles on actuation of the inhaler.

As already stated, the effectiveness of an aerosol device, for example a pressurized metered dose inhaler, is a function of the dose deposited at the appropriate site in the lungs.

Deposition is affected by several factors, one of the most important being the aerodynamic particle size of the particles constituting the aerosol.

Solid particles and/or droplets dispersed in an aerosol formulation can be characterized by their mass median aerodynamic diameter (MMAD), i.e. the diameter around which the mass of the particles is distributed equally.

Particle deposition in the lung largely depends on three physical mechanisms: (1) impaction, a function of particle inertia; (2) sedimentation due to gravity; and (3) diffusion resulting from Brownian motion of fine, submicrometer (< 1 µm) particles.

The mass of the particles determines which of the three main mechanisms predominates.

The effective aerodynamic diameter is a function of the size, shape and density of the particles and will affect the magnitude of the forces acting on them. For example, while inertial and gravitational effects increase with increasing particle size, the displacements produced by diffusion decrease. Therefore, the MMAD of the aerosol particles is particularly important for deposition of the particles in the respiratory tract. GSD (Geometric Standard Deviation) is a measure of the variability of the particle diameters in the aerosol.

Aerosol particles of equivalent aerodynamic size have similar deposition in the lung, irrespective of their effective size and composition.

Particles with aerodynamic diameter of about 0.8 to 5 µm are usually considered respirable.

Particles which are larger than 5 µm in diameter are primarily deposited by inertial impaction in the oropharynx, particles 0.5 to 5 µm in diameter, influenced mainly by gravity, are ideal for deposition in the respiratory tract, and particles 0.5 to 3 µm in diameter are desirable for aerosol delivery to the lung periphery.

Particles smaller than 0.5 µm may be exhaled.

A further parameter which characterizes the efficacy of a metered aerosol is the fine particle dose (FPD) delivered which provides a direct measurement of the aerosol particles lying within a determined size range.

Particle size analysis of an aerosol is measured according to European Pharmacopoeia Ed. III, 1997 by means of an Andersen Cascade Impactor, a device which retains the aerosol particles depending on the aerodynamic diameter, thus performing the particle size analysis.

Eight stages with different cut-off can be used. The particle size distribution profile is obtained by plotting the weight of the deposited drug in each stage against the corresponding cut-off diameters.

Lewis D A et al reported in Respiratory Drug Delivery, VI, pages 363-364, 1998, that using commercially available actuators for delivering solution formulations of aerosol pressurized with HFA, reduction in the orifice diameter induces an increase in the fine particle dose (FPD), with a small, but statistically insignificant, decrease in mass median aerodynamic diameter (MMAD).

It has now been found that, when using the Jet actuator disclosed in the above mentioned EP-B-0475257 for delivering aerosol formulations in HFA solution, FPD is not affected by the actuator orifice diameter of a range between 0.30 to 0.50 mm. Conversely, it has been observed that, when conventional actuators are coupled with other commercial spacers or reservoirs such as Aerochamber or Volumatic, FPD and MMAD values change compared with those obtained with the simple actuator, but the relationship between actuator orifice diameter and FPD does not change, and also in this case the fine particle dose increases as the actuator orifice diameter decreases.

The MMAD values of the formulations delivered by the Jet device are not significantly different than those delivered with a standard actuator.

A further problem with metered aerosol therapy is that, as the number of shots increases, reduction of the orifice diameter may occur during the product use, due to deposition of coarser particles thereon.

The partial clogging of the actuator orifice when the standard actuator is coupled with a reservoir such as Volumatic induces an increase both in the delivered dose and in the fine particle dose. Therefore, lack of uniformity of the dose occurs along repeated actuations.

It has now been found that the Jet actuator, contrary to the conventional actuators coupled with or without a reservoir or a spacer, allows to achieve reproducible delivered dose as well as FPD along repeated actuations.

The results are shown in the following examples.

### EXAMPLE 1

Cans containing different HFA 134a solution formulations of beclomethasone dipropionate (BDP) in the presence of ethanol and optionally of a low-volatility component, such as glycerol, werefitted with :
- different kind of standard actuators having orifice diameters ranging from 0.25 to 0.50 mm;
- different kind of Jet actuators having orifice diameters ranging from 0.30 to 0.50 mm;
- different kind of standard actuators having orifice diameters ranging from 0.25 to 0.42 mm coupled with Aerochamber and Volumatic.

Particle size distribution and MMAD of the aerosol particles delivered by the different combinations were established by tests carried out with the Andersen Cascade Impactor. In such tests FPD was calculated as the mass of the particles deposited from Stage 3 to Filter, and therefore with aerodynamic diameter less than 4.7 µm.

The results, shown in tables 1 - 6, are the mean of two to six shots for each device.

**Table 1: Fine Particle Dose (FPD) as Function of Standard Actuator Orifice Diameter**

| | | | | | |
|---|---|---|---|---|---|
| Formulation: | BDP | | 50 mg/10 ml (250 µg/dose) | | |
| | Ethanol | | 15% ± 0.5% w/w | | |
| | HFA 134a to 12 ml | | | | |
| Standard actuator orifice diameter (mm) | | 0.25 | 0.33 | 0.42 | 0.50 |
| Delivered dose (µg) | | 206 | 222 | 209 | 210 |
| FPD (µg) | | 105 | 66 | 41 | 33 |
| MMAD (µm) | | 1.4 | 1.8 | 1.7 | 1.8 |
| GSD | | 1.9 | 2.2 | 2.3 | 2.6 |

Delivered dose is the amount of drug delivered from the device recovered in all the stages of the Andersen Cascade Impactor.

FPD is the Fine Particle Dose calculated as the mass of the particles deposited from Stage 3 to Filter, and therefore with aerodynamic diameter less than 4.7 µm.

MMAD is the Mass Median Aerodynamic Diameter, i.e. the diameter around which the mass of the particles is distributed equally.

GSD is the Geometric Standard Deviation, a measure of the variability of the particle diameters in the aerosol.

**Table 2: Fine Particle Dose (FPD) as Function of Standard Actuator Orifice Diameter**

| | | | | | |
|---|---|---|---|---|---|
| Formulation: | BDP | | 50 mg/10 ml (250 µg/dose) | | |
| | Ethanol | | 15% ± 0.5% w/w | | |
| | Glycerol | | 1.3% w/w | | |
| | HFA 134a to 12 ml | | | | |
| Standard actuator orifice diameter (mm) | | 0.25 | 0.30 | 0.33 | 0.42 |
| Delivered dose (µg) | | 221 | 242 | 229 | 216 |
| FPD (µg) | | 94 | 55 | 46 | 35 |
| MMAD(µm) | | 2.6 | 2.6 | 3.0 | 2.9 |
| GSD | | 2.0 | 2.3 | 2.3 | 2.3 |

**Table 3: Fine Particle Dose (FPD) as Function of Jet Actuator Orifice Diameter**

| | | | | | |
|---|---|---|---|---|---|
| Formulation: | BDP | | 50 mg/10 ml (250 µg/dose) | | |
| | Ethanol | | 15% ± 0.5% w/w | | |
| | Glycerol | | 1.3% w/w | | |
| | HFA 134a to 12 ml | | | | |
| Jet Actuator Orifice Diameter (mm) | | 0.30 | 0.35 | 0.40 | 0.45 |
| Delivered dose (µg) | | 76 | 76 | 80 | 77 |
| FPD (µg) | | 58 | 57 | 53 | 55 |
| MMAD (µm) | | 2.3 | 2.4 | 2.7 | 2.5 |
| GSD | | 2.0 | 1.9 | 2.0 | 1.9 |

**Table 4: Fine Particle Dose (FPD) as Function of Jet Actuator Orifice Diameter**

| | | | | | |
|---|---|---|---|---|---|
| Formulation: | BDP | | 50 mg/10 ml (250 µg/dose) | | |
| | Ethanol | | 15% ± 0.5% w/w | | |
| | HFA 134a to 12 ml | | | | |
| Jet Actuator Orifice Diameter (mm) | 0.30 | 0.35 | 0.40 | 0.45 | 0.50 |
| Delivered dose (µg) | 77 | 81 | 79 | 72 | 74 |
| FPD (µg) | 67 | 71 | 62 | 59 | 59 |
| MMAD (µm) | 1.4 | 1.5 | 1.6 | 1.7 | 1.7 |
| GSD | 2.1 | 2.1 | 2.1 | 2.0 | 2.0 |

**Table 5: Fine Particle Dose (FPD) as Function of Standard Actuator Orifice Diameter coupled with Aerochamber**

| | | | | |
|---|---|---|---|---|
| Formulation: | BDP | | 50 mg/10 ml (250 µg/dose) | |
| | Ethanol | | 15% ± 0.5% w/w | |
| | Glycerol | | 1.3% w/w | |
| | HFA 134a to 12 ml | | | |
| Standard actuator orifice diameter (mm) | | 0.25 | 0.30 | 0.42 |
| Delivered dose (µg) | | 114 | 96 | 74 |
| FPD (µg) | | 82 | 68 | 45 |
| MMAD (µm) | | 2.6 | 2.5 | 2.6 |
| GSD | | 1.9 | 1.9 | 1.9 |

It can be observed that a significant increase of FPD occurs with the reduction of the orifice diameter.

**Table 6: Fine Particle Dose (FPD) as Function of Standard Actuator Orifice Diameter coupled with Volumatic**

| | | | | |
|---|---|---|---|---|
| Formulation: | BDP | 50 mg/10 ml (250 µg/dose) | | |
| | Ethanol | 15% ± 0.5% w/w | | |
| | Glycerol | 1.3% w/w | | |
| | HFA 134a to 12 ml | | | |
| Standard actuator orifice diameter (mm) | | 0.25 | 0.30 | 0.42 |
| Delivered dose (µg) | | 169 | 132 | 118 |
| FPD (µg) | | 120 | 87 | 74 |
| MMAD (µm) | | 3.1 | 3.3 | 3.5 |
| GSD | | 1.6 | 1.6 | 1.8 |

Also in this case a significant increase of FPD occurs with the reduction of the orifice diameter.

### EXAMPLE 2

Metered cans containing the same HFA 134a solution formulation of beclomethasone (BDP) were fitted with:
- Different kind of standard actuators having orifice diameters ranging from 0.30 to 0.42 mm coupled with Aerochamber and Volumatic spacers;
- Jet actuator having orifice diameter of 0.40 mm.

The delivered dose and FPD were evaluated at increasing numbers of shots. The delivered dose and FPD values are the mean of ten shots. The particle size distribution and MMAD of the aerosol particles delivered by the different combinations were established by tests carried out with the Andersen Cascade Impactor.

The results, shown in Table 7, confirm that, along repeated actuations, a significant increase of FPD occurs with the reduction of the diameter of the orifice.

In the case of Volumatic, along repeated actuations, an increase both in the delivered dose and in the fine particle dose is also evident.

**Table 7: Comparison of the effect of Jet, Aerochamber and Volumatic Actuators on the delivered dose and fine particle dose (FPD) after repeated actuations.**

| | | | | | | |
|---|---|---|---|---|---|---|
| Formulation | BDP | 50 mg/10 ml (250 µg/dose) | | | | |
| | Ethanol | 15% ± 0.5% w/w | | | | |
| | Glycerol | 1.3% w/w | | | | |
| | HFA 134a to 12 ml | | | | | |

| ACTUATOR | CAN AND ACTUATOR DIAMETERS (mm) | NUMBER OF SHOTS | DELIVERED DOSE (µg) | FPD (µg) | MMAD (µm) | GSD |
|---|---|---|---|---|---|---|
| JET | B (0.40) | 21-30 | 93 | 65 | 2.8 | 1.9 |
| | B (0.40) | 141-150 | 85 | 51 | 3.0 | 1.9 |
| | | | | | | |
| | C (0.40) | 55-65 | 95 | 58 | 3.0 | 1.9 |
| | C (0.40) | 161-170 | 96 | 57 | 3.0 | 1.9 |
| AEROCHAMBER | B (0.30) | 36-45 | n.a. | 68 | 2.5 | 1.9 |
| | C (0.30) | 146-155 | 86 | 68 | 2.8 | 1.8 |
| | | | | | | |
| | B (0.42) | 111-120 | 56 | 45 | 2.6 | 1.9 |
| | C (0.42) | 86-95 | 59 | 47 | 2.7 | 1.9 |
| VOLUMATIC | B (0.30) | 81-90 | 118 | 87 | 3.3 | 1.6 |
| | C (0.30) | 131-140 | 88 | 67 | 3.1 | 1.7 |
| | | | | | | |
| | B (0.42) | 126-135 | 108 | 74 | 3.5 | 1.6 |
| | C (0.42) | 71-80 | 74 | 56 | 3.2 | 1.6 |

| | | | | | | |
|---|---|---|---|---|---|---|
| n.a. = not available | | | | | | |

## Claims

1. A device for use with a pressurized can for delivering metered aerosol formulations, said device comprising a flat body with a seat for housing the can, an inhalation mouthpiece and an expansion chamber shaped to create a vortex flow of the aerosol particles expelled by an actuator orifice, wherein the delivered aerosol formulations comprise an active ingredient in solution in a propellant consisting of a hydrofluoroalkane (HFA) selected from 1,1,1,2-tetrafluoroethane (HFA 134a), 1,1,1,2,3,3,3-heptafluoropropane (HFA 227) or mixtures thereof, a co-solvent such as ethanol and optionally a low-volatility component preferably selected from glycerol, propylene glycol, polyethylene glycol, oleic acid and isopropyl myristate, and the actuator orifice diameter is in the range between 0.30 and 0.50 mm.

## Patentansprüche

1. Vorrichtung zur Verwendung mit einem druckbeaufschlagten Behälter zum Abgeben von dosierten Aerosolformulierungen, wobei die Vorrichtung einen flachen Körper mit einem Sitz zur Aufnahme des Behälters, ein Inhalationsmundstück und eine Expansionskammer, die derart geformt ist, um eine Wirbelströmung der von einer Sprühkopföffnung ausgestoßenen Aerosolteilchen zu bewirken, umfasst, wobei die abgegebenen Aerosolformulierungen einen Wirkstoff in Lösung in einem Treibmittel, bestehend aus einem Hydrofluoralkan (HFA) ausgewählt aus 1,1,1,2-Tetrafluorethan (HFA 134a), 1,1,1,2,3,3,3-Heptafluorpropan (HFA 227) oder Gemischen davon, ein Cosolvens, wie Ethanol, und gegebenenfalls eine schwerflüchtige Komponente, vorzugsweise ausgewählt aus Glycerin, Propylenglykol, Polyethylenglykol, Ölsäure und Isopropylmyristat, umfassen und der Durchmesser der Sprühkopföffnung in dem Bereich zwischen 0,30 und 0,50 mm liegt.

## Revendications

1. Dispositif pour utilisation avec une bombe sous pression pour délivrer des formulations d'aérosol de manière dosée, ledit dispositif comprenant un corps plat pourvu d'un logement pour recevoir la bombe, un embout buccal pour l'inhalation et une chambre d'expansion configurée pour créer un écoulement tourbillonnaire des particules d'aérosol expulsées par un orifice d'un élément de commande, dans lequel les formations d'aérosol délivrées comprennent un ingrédient actif en solution dans un propulseur constitué d'un hydrofluoroalcane (HFA) choisi parmi le 1,1,1,2-tétrafluoroéthane (HFA 134a), le 1,1,1,2,3,3,3-heptafluoropropane (HFA 227) et leurs mélanges, un co-solvant tel que l'éthanol et éventuellement un composant de faible volatilité de préférence choisi parmi le glycérol, le propylène glycol, le polyéthylène glycol, l'acide oléique et le myristate d'isopropyle, et le diamètre de l'orifice de l'élément de commande est dans la plage comprise entre 0,30 et 0,50 mm.
